(19) **Europäisches Patentamt / European Patent Office / Office européen des brevets**

(11) **EP 3 734 254 B1**

(12) **EUROPEAN PATENT SPECIFICATION**

(45) Date of publication and mention of the grant of the patent:
**22.06.2022 Bulletin 2022/25**

(21) Application number: **20170982.1**

(22) Date of filing: **23.04.2020**

(51) International Patent Classification (IPC):
**G01N 21/35** (2014.01)     **G01N 33/02** (2006.01)

(52) Cooperative Patent Classification (CPC):
**G01N 21/3563; G01N 21/359; G01N 33/02;**
G01N 2001/2866; G01N 2001/386;
G01N 2001/4083; G01N 2201/129

(54) **METHOD FOR DETERMINING WATER SOLUBLE PROTEIN CONTENT (WSPC) IN SOYBEAN BY NEAR INFRARED SPECTROSCOPY (NIRS)**

VERFAHREN ZUR BESTIMMUNG DES GEHALTS AN WASSERLÖSLICHEN PROTEINEN (WSPC) IN SOJABOHNEN DURCH NAHINFRAROTSPEKTROSKOPIE (NIRS)

PROCÉDÉ DE DÉTERMINATION D'UN CONTENU DE PROTÉINES SOLUBLES DANS L'EAU (WSPC) DANS DES GRAINES DE SOJA PAR SPECTROSCOPIE INFRAROUGE PROCHE (NIRS)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.04.2019 CN 201910339010**

(43) Date of publication of application:
**04.11.2020 Bulletin 2020/45**

(73) Proprietors:
• **Institute of Crop Sciences, Chinese Academy of Agricultural Sciences**
  **Beijing 100081 (CN)**
• **Yangtze University**
  **Jingzhou, Hubei 434023 (CN)**

(72) Inventors:
• **QIU, Lijuan**
  **BEIJING (CN)**
• **WANG, Jun**
  **BEIJING (CN)**
• **XU, Ruixin**
  **BEIJING (CN)**
• **SONG, Jian**
  **BEIJING (CN)**

(74) Representative: **Hirsch & Associés**
**154 Boulevard Haussmann**
**75008 Paris (FR)**

(56) References cited:
• **YAKUN ZHANG ET AL: "Rapid determination of soluble protein content for soybean leaves based on near infrared spectroscopy", NONGYE GONGCHENG XUEBAO = TRANSACTIONS CHINESE SOCIETY OF AGRICULTURAL ENGINEERING, vol. 34, 15 September 2018 (2018-09-15), pages 187-193, XP055733975, CN ISSN: 1002-6819, DOI: 10.11975/j.issn.1002-6819.2018.18.023**
• **WEIGUO LU ET AL: "Identification of the quantitative trait loci (QTL) underlying water soluble protein content in soybean", THEORETICAL AND APPLIED GENETICS ; INTERNATIONAL JOURNAL OF PLANT BREEDING RESEARCH, vol. 126, no. 2, 7 October 2012 (2012-10-07), pages 425-433, XP055734277, Berlin, DE ISSN: 0040-5752, DOI: 10.1007/s00122-012-1990-8**
• **WANG JINSHUI ET AL: "Measurement of protein content in sesame by near-infrared spectroscopy technique", NEW TECHNOLOGY OF AGRICULTURAL ENGINEERING (ICAE), 2011 INTERNATIONAL CONFERENCE ON, IEEE, 27 May 2011 (2011-05-27), pages 1092-1095, XP031955814, DOI: 10.1109/ICAE.2011.5943978 ISBN: 978-1-4244-9574-0**

EP 3 734 254 B1

- XU RUIXIN ET AL: "Use of near-infrared spectroscopy for the rapid evaluation of soybean [Glycine max (L.) Merri.] water soluble protein content", SPECTROCHIMICA ACTA. PART A: MOLECULAR AND BIOMOLECULAR SPECTROSCOPY, ELSEVIER, AMSTERDAM, NL, vol. 224, 14 August 2019 (2019-08-14), XP085864144, ISSN: 1386-1425, DOI: 10.1016/J.SAA.2019.117400 [retrieved on 2019-08-14]
- Baianu I. ET AL: "Determination of Soybean Oil, Protein and Amino Acid Residues in Soybean Seeds by High Resolution Nuclear Magnetic Resonance (NMRS) and Near Infrared (NIRS).", Nature Precedings, 2 April 2012 (2012-04-02), XP055805621, DOI: 10.1038/npre.2012.7053.1 Retrieved from the Internet: URL:http://www.nature.com/articles/npre.20 12.7053.1>

**Description**

## TECHNICAL FIELD

The present invention relates to the field of near infrared spectroscopy (NIRS), and in particular, to a method for determining a water soluble protein content (WSPC) in soybean by NIRS.

## BACKGROUND

Soybean seeds are rich in a variety of nutrients, including high-quality protein, lipid, isoflavone, vitamin E, etc. and especially have a protein content of 40%, which is higher than other oil and grain crops. The soybean seeds are known as "vegetarian meat", "meat from dry fields", etc. Soy protein can be classified into storage protein, enzyme and physiologically active protein according to physiological function. The storage protein can be divided into globulin and albumin according to structure and dissolution characteristics. The globulin accounts for about 90%, soluble globulin accounts for 59%-81%, and insoluble globulin accounts for about 3%-7%. The soy protein is rich in eight essential amino acids required by human beings, which are similar to meat amino acids. Except sulfur-containing amino acids (cysteine and methionine), other nutritional components are relatively balanced and easy to absorb. Water soluble protein of soybean is very important to the processing functional characteristics of soybean, which directly affects the processing quality of soybean. The water soluble protein in soybean accounts for about 90% of the total protein of soybean, of which about 86% precipitates in a solution with pH of 4-5. Calcium phytate, magnesium and salts in soybean can combine with protein under acidic conditions to form precipitates at pH 4.2. Therefore, it is believed that an isoelectric point of soybean protein has a pH of about 4.2, and the soybean protein without isoelectric precipitation in soybean protein is whey protein (low molecular proteins such as various enzymes and amino acids), which accounts for about 6%-7% of the total soybean.

NIRS is the fastest growing and most noticeable technology since the 1990s. It is the organic combination of a spectral measurement technology and chemometrics, and is known as the "giant of analysis". A near infrared spectrum is generated when molecular vibration transition is performed from a ground state to a high energy level due to non-resonance of molecular vibration. Two technologies are mainly used to obtain the near infrared spectrum: a transmission spectrum technology and a reflection spectrum technology. In the near infrared spectrum analysis, the near infrared spectrum of a tested substance depends on the composition and structure of the sample. There is a certain functional relationship between the composition and structure of the sample and the near infrared spectrum. Chemometrics is used to determine these important functional relationships; that is, after correction, various data can be quickly calculated according to the near infrared spectrum of the tested sample. The commonly used correction methods mainly include: multiple linear regression (MLR), principal component analysis (PCA), partial least squares (PLS), artificial neural network (ANN), a topological method, etc. Through the combination of the chemometrics and the NIRS, spectral curves of crude fat, crude protein, crude fatty acid, moisture, etc. of soybean have been successfully constructed, and these indexes are rapidly determined.

Water soluble protein of soybean is very important to the processing functional characteristics of soybean, which directly affects the processing quality of soybean. Accurate and rapid measurement of the WSPC in soybean is of great significance for selecting high-quality processed soybean varieties. At present, methods for measuring the WSPC in soybean mainly include a chemical determination method and an NIRS. The chemical determination method is mainly based on the 2007 implementation standard of the Ministry of Agriculture of the PRC: NY/T 1205-2006 Determination of the WSPC in Soybean. Although this method can accurately measure the WSPC in soybean, the experimental steps are complex and the measurement cycle is long, so that the WSPC in soybean cannot be quickly measured. The NIRS mainly uses the combination of spectral measurement technology and chemometrics to construct a spectral model to measure the WSPC in soybean. The currently constructed model for predicting a WSPC in soybean can only predict the WSPC in soybean with yellow seed coat, but cannot eliminate the influence of the seed coat color on the spectrum. It cannot accurately measure the WSPC in soybean with other seed coat colors, such as black, green and brown, and the predicted content is inaccurate and lower than the actual WSPC.

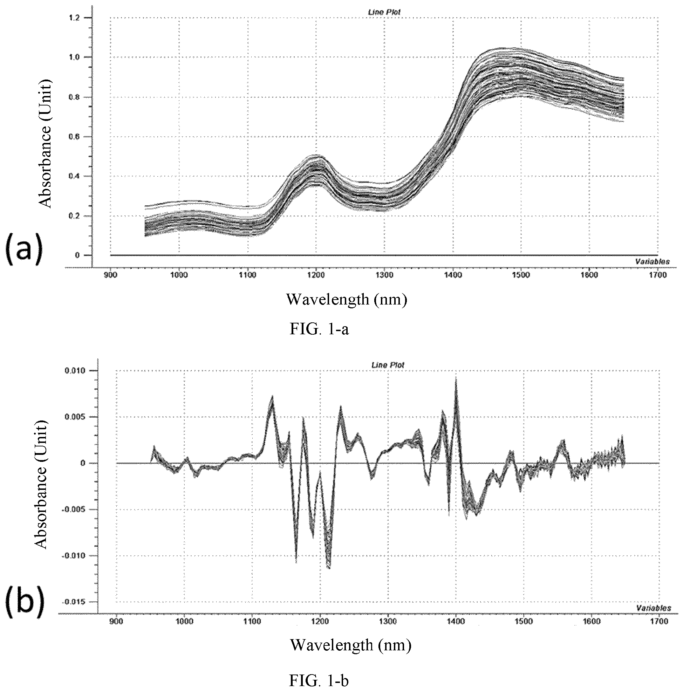

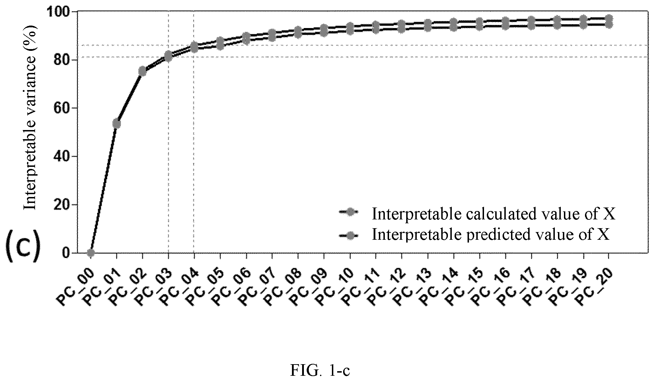

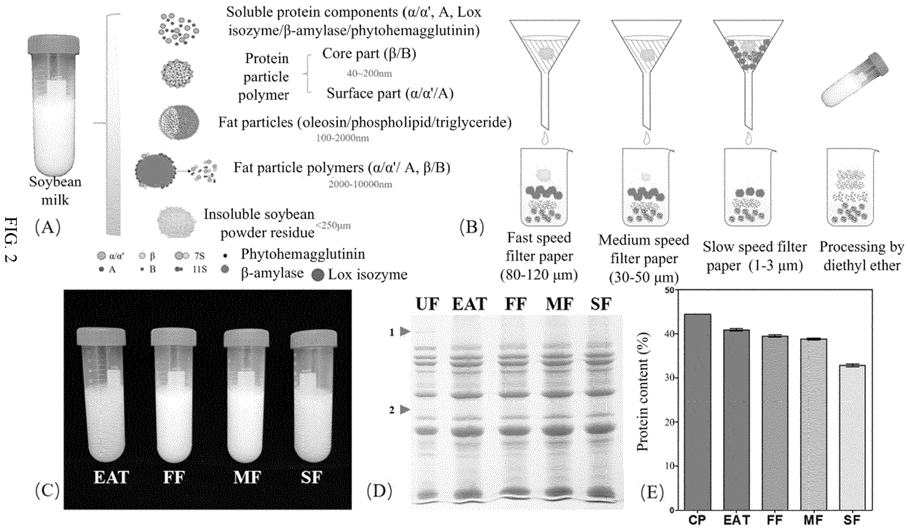

Yakun Zhang ET AL: "Rapid determination of soluble protein content for soybean leaves based on near infrared spectroscopy", Nongye Gongcheng Xuebao = Transactions of the Chinese Society of Agricultural Engineering, vol. 34, 15 September 2018 (2018-09-15), pages 187-193, XP055733975, CN ISSN: 1002-6819, DOI: 10.11975/j.issn.1002-6819.2018.18.023 discloses a method for determining a water soluble protein content (WSPC, cf., e.g., the abstract) in soybean leaves by near infrared spectroscopy (NIRS), comprising the following steps: scanning the soybean leaves with a near infrared spectrometer to obtain an original spectrum, wherein the wavelength range of the original spectrum is 1100-2200 nm (cf. Figs. 1,2); and substituting the original spectrum of the soybean into a model for predicting a WSPC in soybean to obtain the WSPC in soybean (cf. section 1.6, p. 189); wherein the construction of the model for predicting a WSPC in soybean comprises:

S1: processing each soybean sample to obtain an original spectrum (cf. Fig. 1);

S2: determining the WSPC by a chemical method, and recording the content as a measured value (cf. section 1.3, p. 188);

S3: preprocessing the original spectrum obtained in step S1 by standard normal variate method (SNV), multiple scattering correction (MSC) and Sabitzky-Golay fitting method (SG) respectively or in combination (cf. section 1.4, p. 188), and establishing a quantitative analysis model by performing partial least squares regression on the pre-processed spectrum (cf. section 1.6, p. 189);

S4: using the quantitative analysis model established in step S3 to test the soybean sample, and recording an obtained value as a predicted value (as described in section 2.3 on page 190); and

S5: by comparing the predicted value with the measured value (as defined in section 1.3, p. 188, the measured value is used as a reference value), calculating a standard root mean square error (RMSE) and a ratio of prediction to deviation of various quantitative analysis models (see section 2.3, p. 190) that may be established in step S3, and selecting an optimal standard RMSE and ratio of prediction to deviation of the various quantitative analysis models to obtain the model for predicting a WSPC in soybean (see section 2.3 and Table 3, p. 190; see also Figs. 4 and 5 on page 191).

Weiguo Lu ET AL: "Identification of the quantitative trait loci (QTL) underlying water soluble protein content in soybean", Theoretical and Applied Genetics ; International Journal of Plant Breeding Research, vol. 126, no. 2, 7 October 2012 (2012-10-07), pages 425-433, XP055734277, Berlin, DE ISSN: 0040-5752, DOI: 10.1007/s00122-012-1990-8 describes determination of WSPC in soybean by a chemical method. Section "Measurement of water soluble protein" (p. 426-427) describes similar process steps for the same purpose.

WANG JINSHUI ET AL: "Measurement of protein content in sesame by near-infrared spectroscopy technique", NEW TECHNOLOGY OF AGRICULTURAL ENGINEERING (ICAE), 2011 INTERNATIONAL CONFERENCE ON, IEEE, 27 May 2011 (2011-05-27), pages 1092-1095, XP031955814, DOI: 10.1109/ICAE.2011.5943978, ISBN: 978-1-4244-9574-0 describes a model of determining sesame protein using near infrared spectroscopy (NIRS) and a FOSS system as the analyzer. The influences on the model of factors, such as the mathematics methods and optics treatment methods were studied. The results of model validation showed that the best factors were SNV only for optics treatment method and "3, 3, 3, 1" for mathematics method. The average determination coefficient of validation (RSQ) was 0.9826, the square error of cross (SEC) was 0.2313, the correlation coefficient (1-VR) was 0.7272, the square error of cross validation (SECV) was 0.9134, the average determination coefficient of validation (RSQ) was 0.896, and the standard error of prediction (SEP) was 0.827. This model could determine the protein content in sesame used as a rapid method to detect the quality of sesame seeds.

Baianu I. ET AL: "Determination of Soybean Oil, Protein and Amino Acid Residues in Soybean Seeds by High Resolution Nuclear Magnetic Resonance (NMRS) and Near Infrared (NIRS).", Nature Precedings, 2 April 2012 (2012-04-02), XP055805621, DOI: 10.1038/npre.2012.7053.1, URL:http://www.nature.com/articles/npre.2012.7053.1. (Sections 3.1-3.3, 3.6, 3.7 and 4.1) discloses NIRS measurements of whole soybean seeds using MSC spectra preprocessing and a partial least squares model for comparing a NIRS prediction model to standard protein values to obtain a model for predicting protein content.

## SUMMARY

In order to overcome the shortcoming of inaccurate content determination of an existing method for spectrum detection of a WSPC in soybean, the present invention provides a method for determining a WSPC in soybean by NIRS, which effectively improves the accuracy in determining the WSPC in soybean, and makes the determination more convenient and accurate.

To achieve the above purpose, the present invention provides the following technical solution.

A method for determining a WSPC in soybean by NIRS includes the following steps:

(1): peeling off seed coat of to-be-tested soybean to obtain peeled soybean;

(2): scanning the peeled soybean with a near infrared spectrometer to obtain an original spectrum, where the wavelength range of the original spectrum is 950-1650 nm; and

(3): substituting the original spectrum of the soybean into a model for predicting a WSPC in soybean to obtain the WSPC in soybean;

where the construction of the model for predicting a WSPC in soybean includes:

S1: processing each soybean sample according to steps (1) to (2) to obtain an original spectrum;

S2: defatting the soybean sample processed in step S1, determining the WSPC by a chemical method, and recording the content as a measured value; wherein the defatting comprises: crushing the scanned peeled soybean sample, shaking and mixing with diethyl ether, centrifuging to remove a supernatant, and volatilizing the diethyl ether to obtain the defatted soybean sample; and the chemical method for determining the WSPC in soybean comprises the following steps:

A1: mixing the defatted soybean sample with water, performing shaking extraction, and centrifuging to obtain a supernatant to obtain a water soluble protein extract; and
A2: using a Kjeldahl nitrogen determining method to determine the content of protein in the water soluble protein extract to obtain the WSPC in the soybean sample, and recording the content as a measured value;

S3: preprocessing the original spectrum obtained in step S1 by MSC+SG, and establishing a quantitative analysis model by performing partial least squares regression on the preprocessed spectrum;
S4: using the quantitative analysis model established in step S3 to test the soybean sample, and recording an obtained value as a predicted value; and
S5: by comparing the predicted value with the measured value, calculating a standard root mean square error (RMSE) and a correlation coefficient of various quantitative analysis models that may be established in step S3, and selecting an optimal standard RMSE and correlation coefficient of the various quantitative analysis models to obtain the model for predicting a WSPC in soybean.

Preferably, in step (2), the peeled soybean is repeatedly scanned three times, and an average spectrum is calculated as the original spectrum.
Preferably, in step S2, a ratio of the weight of the peeled soybean to the volume of the diethyl ether is (2-10) g:(30-80) ml.
Preferably, in step S2, a shaking and mixing speed is 180-300 r/min, and the shaking and mixing time is 20-40 min.
Preferably, in step S2, a centrifuging speed is 1500-2500 r/min and the centrifuging time is 8-15 min.
Preferably, in step A1, the operation of mixing with water until centrifugation is implemented is repeated 2-3 times, and the obtained supernatants are combined to obtain the water soluble protein extract.
Compared with the prior art, the present invention has the following beneficial effects:
The present invention provides a method for determining a WSPC in soybean by NIRS. The method includes peeling off seed coat of to-be-tested soybean first to eliminate the influence of the soybean seed coat color on NIRS; scanning the peeled soybean with a near infrared spectrometer to obtain an original spectrum, where the wavelength range of the original spectrum is 950-1650 nm; and substituting the original spectrum of the soybean into a model for predicting a WSPC in soybean to obtain the WSPC in soybean. When the model for predicting a WSPC in soybean is constructed, the soybean is peeled, defatted and determined by a chemical method, the original spectrum is preprocessed by a plurality of methods, and a measured value and a predicted value are compared to evaluate a quantitative analysis model obtained by a PLS regression method, and finally the model for predicting a WSPC in soybean with high prediction accuracy is obtained.

(1) The method provided by the present invention effectively solves the measurement error caused by the color difference of seed coat when the content of effective substances in soybean is detected by conventional spectrometry.
(2) The method for extracting water soluble protein in industry standard fails to extract water soluble protein from soybean fat particles, which results in low WSPC in soybean. Fat particles adsorb some water soluble protein components, so that some water soluble protein cannot be normally soluble in water. Therefore, when the model for predicting a WSPC in soybean is constructed according to the present invention, the peeled soybean is defatted first to separate the fat particles from the water soluble protein, so that the water soluble protein is fully soluble in water to obtain a more accurate measured value by a chemical method, and a more accurate model for predicting a WSPC in soybean is constructed by using the measured value.
(3) The method according to the present invention does not perform destructive processing on soybean, results can be obtained only by spectral scanning, and thus the method is simple, convenient and efficient.

## BRIEF DESCRIPTION OF THE DRAWINGS

FIG. 1 is a schematic diagram of near infrared spectrum acquisition and PLS modeling, where a is an original spectrum; b is a spectrum preprocessed by MSC+SNV+SG; and c shows the principal component analysis of the preprocessed spectrum;

FIG. 2 shows the influence of different defatting methods on the content and components of water soluble protein in soybean, where A is the particle composition in raw soybean milk; B is a schematic diagram of different defatting methods; C shows the clarity comparison of water soluble protein solution; D shows an SDS-PAGE test result of the water soluble protein extraction solution; and E shows the contents of water soluble protein obtained by defatting by different methods;

in FIG. 2, UF refers to a control group without defatting processing such as filtration, and the obtained water soluble protein solution is a suspension, containing soybean powder particles, which contain water soluble protein and affect the test results;

EAT represents defatting processing by diethyl ether, which removes fat particles, and does not filter out macromolecular protein particles while releasing fat particle related protein;

FF represents filtration with fast speed filter paper, where the filter paper has a pore diameter of 80-120 $\mu$m and can filter out soybean powder particles with a particle size greater than 120$\mu$m;

MF represents filtration with medium speed filter paper, where the filter paper has a pore diameter of 30-50 $\mu$m;

SF represents filtration with slow speed filter paper, where the filter paper has a pore diameter of 1-3 $\mu$m;

CP represents the total protein content of a sample;

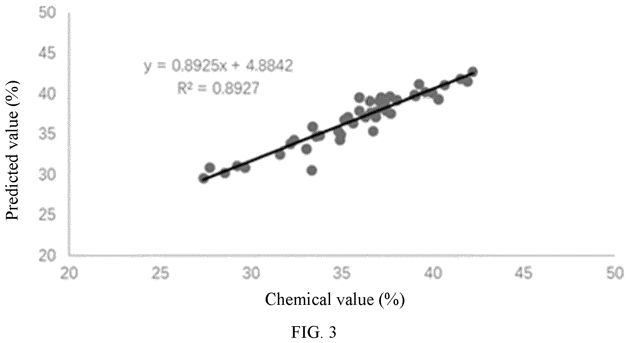

FIG. 3 is a scatter diagram of a correlation between chemical values and predicted values of a near infrared model of water soluble protein in soybean in Embodiment 3.

## DETAILED DESCRIPTION

The present invention provides a method for determining a WSPC in soybean by NIRS, including the following steps:

(1): Peel off seed coat of to-be-tested soybean to obtain peeled soybean.
(2): Scan the peeled soybean with a near infrared spectrometer to obtain an original spectrum, where the wavelength range of the original spectrum is 950-1650 nm.
(3): Substitute the original spectrum of the soybean into a model for predicting a WSPC in soybean to obtain the WSPC in soybean.

The construction of the model for predicting a WSPC in soybean includes:

S1: Process each soybean sample according to steps (1) to (2) to obtain an original spectrum.
S2: Defat the soybean sample processed in step S1, determine the WSPC by a chemical method, and record the content as a measured value.
S3: Preprocess the original spectrum obtained in step S1 by MSC+SG, and establish a quantitative analysis model by performing partial least squares regression on the preprocessed spectrum.
S4: Use the quantitative analysis model established in step S3 to test the soybean sample, and recording an obtained value as a predicted value.
S5: By comparing the predicted value with the measured value, calculate a standard RMSE and a correlation coefficient of various quantitative analysis models that may be established in step S3, and select an optimal standard RMSE and correlation coefficient of the various quantitative analysis models to obtain the model for predicting a WSPC in soybean.

In the present invention, seed coat of to-be-tested soybean is peeled off first to obtain peeled soybean. The colors of peeled soybean are mainly yellow and green. The influence of different soybean seed coat colors on NIRS results is eliminated and the test accuracy is improved.
After the peeled soybean is obtained, in the present invention, the peeled soybean is scanned with a near infrared spectrometer to obtain an original spectrum, where the wavelength range of the original spectrum is 950-1650 nm. In the present invention, the peeled soybean is preferably scanned three times repeatedly, and an average spectrum is calculated as the original spectrum to eliminate the influence of primers such as sample particle size and uniformity on the spectral scanning results. In the present invention, the original spectrum can be directly used without preprocessing during determination, which is simple and convenient.

After the original spectrum is obtained, in the present invention, the original spectrum of the soybean is substituted into a model for predicting a WSPC in soybean to obtain the WSPC in soybean. The model for predicting a WSPC in soybean constructed according to the present invention has accurate prediction and high efficiency. Specifically, where the construction of the model for predicting a WSPC in soybean including the following steps:

S1: After the soybean sample is peeled to obtain the peeled soybean sample, scan the peeled soybean sample with a near infrared spectrometer to obtain an original spectrum, where the wavelength range of the original spectrum is 950-1650 nm.

S2: Defat the scanned peeled soybean sample, determine the WSPC by a chemical method, and record the content as a measured value.

In the present invention, the defatting method includes: crushing the peeled soybean sample, mixing the crushed peeled soybean sample with diethyl ether and shaking, centrifuging to remove a supernatant, and volatilizing the diethyl ether from the obtained precipitate to obtain the defatted soybean sample. In the present invention, the crushing particle size is preferably 40-100 meshes, more preferably 60 meshes. In the present invention, a ratio of the weight of the peeled soybean to the volume of the diethyl ether is (2-10) g:(30-80) ml, more preferably 5 g:50 ml. In the present invention, a mixing and shaking speed is preferably 150-300 r/min, more preferably 220 r/min. In the present invention, the mixing and shaking time is preferably 20-40 min, more preferably 30 min. In the present invention, a centrifuging speed is preferably 1500-2500 r/min, more preferably 2000 r/min. In the present invention, the centrifuging time is preferably 8-15 min, more preferably 10 min. According to the present invention, the purpose of crushing the peeled soybean sample is to better defat the sample. As shown in the specific implementation of the present invention, diethyl ether is the best solvent for soybean defatting. In the present invention, the purpose of defatting the peeled soybean sample is to separate fat particles from water soluble protein so as to obtain a more accurate measured value.

In the present invention, the chemical method for determining the WSPC preferably includes the following steps:

A1: Mix the defatted soybean sample with water, perform shaking extraction, and centrifuge to obtain a supernatant to obtain a water soluble protein extract.

A2: Use a Kjeldahl nitrogen determining method to determine the content of protein in the water soluble protein extract to obtain the WSPC in the soybean sample, and record the content as a measured value.

In the present invention, a temperature during the chemical extraction is 23-27°C, preferably 25°C. In the present invention, a ratio of the weight of the defatted soybean sample to the volume of the water is preferably (2-10) g:(30-80) ml, more preferably 5 g:50 ml. In the present invention, the shaking extraction time is preferably 50-80 min, more preferably 60 min. In the present invention, a shaking extraction speed is preferably 150-300 r/min, more preferably 220 r/min. In the present invention, the centrifuging time is preferably 8-15 min, more preferably 10 min. In the present invention, a centrifuging speed is preferably 500-1500 r/min, more preferably 1000 r/min.

According to the present invention, in order to obtain more accurate determining results, the operation of mixing with water until centrifugation is implemented in step A1 is repeated 2-3 times, and the obtained supernatants are combined to obtain the water soluble protein extract.

In the present invention, the WSPC in the soybean sample is determined according to the ratio of the protein content determined by the Kjeldahl nitrogen determining method to the weight of the soybean sample. The Kjeldahl nitrogen determining method of the present invention can be performed according to conventional methods in the art, and no particular restriction is imposed thereto in the present invention.

S3: Preprocess the original spectrum obtained in step S1 by MSC+SG, and establish a quantitative analysis model by performing partial least squares regression on the preprocessed spectrum.

In the present invention, the SNV refers to a standard normal variate method, the MSC refers to a multiplicative scatter correction method, and the SG refers to a Sabitzky-Golay fitting method. The preprocessing the original spectrum is by MSC+SG.; and then the quantitative analysis model constructed by the PLS regression method obtained by various preprocessing methods is obtained. According to the present invention, MSG+SG are adopted to preprocess the original spectrum, which is beneficial to eliminating baseline, noise and other spectrum variation sources.

S4: Use the quantitative analysis model established in step S3 to test the soybean sample, and record an obtained value as a predicted value.

S5: By comparing the predicted value with the measured value, calculate a standard RMSE and a correlation coefficient of various quantitative analysis models that may be established in step S3, and select an optimal standard RMSE (SEP value) and correlation coefficient ($R^2$) of the various quantitative analysis models to obtain the model for predicting a WSPC in soybean.

The technical solutions provided by the present invention are described in detail below with reference to the embodiments, but the technical solutions cannot be understood as limiting the protection scope of the present invention.

Embodiment 1 Establishment of a model for a near infrared spectrum of water soluble protein in soybean

1. Acquisition and analysis of spectral data of soybean samples

The instrument used in the test was a DA7200 near infrared spectrometer purchased from Swiss Perten Instruments Co., Ltd.

186 soybean samples were selected in this test. Seed coat of the soybean samples was removed respectively, and the peeled soybean samples were placed in a sample rotating tank with a diameter of 75 mm. Each sample was repeatedly loaded and scanned three times to eliminate the influence of factors such as sample particle size and uniformity on the spectrum. An instrument program automatically converted the reflection spectrum information into absorbance values for storage. Unscrambler 9.8 software of the Perten Instruments Co., Ltd was used to preprocess the spectrum and calculate the average spectrum.

2. Extraction of water soluble protein in soybean

The peeled soybean was crushed and sieved with a 60-mesh sieve; 5 g of soybean powder (0.0001 g) was weighed and evenly mixed with 50 ml of diethyl ether, and the mixed solution was continuously shaken for 30 min at 220 r/min to dissolve fat fully. Then centrifugation was performed for 10 min at 2000 r/min, a supernatant was removed, and the solution was placed in a fume cupboard for 30 min until diethyl ether was volatilized completely to obtain the defatted soybean sample.

The water soluble protein was extracted by water solution at room temperature. 50 ml of ultrapure water was added to the defatted soybean sample, and the sample was shaken for 60 min (at $25\pm2°C$) and centrifuged at 1000 r/min for 10 min. Then the supernatant was poured into a 250 ml volumetric flask, and extraction was repeated three times to obtain the water soluble protein extract of soybean. Water was added into a volumetric flask for a constant volume to obtain to-be-tested liquid.

Determination of the WSPC by the kjeldahl nitrogen determining method was performed as follows: 2 g of $CuSO_4\cdot5H_2O$ and $K_2SO_4$ mixed catalyst, 10 ml of to-be-tested liquid and 10 ml of concentrated sulfuric acid were sequentially added into a 500 ml digestion tube. The digestion tube was placed in the fume cupboard for heating and digestion, and was heated at a low temperature of 100°C for 0.5 h until black foam disappeared, and the volume of foam in the bottle was controlled not to exceed 2/3 that of the tube; then the temperature was increased to 420°C and heating was performed for 1.5 h; when the digestive fluid was transparent and blue-green, the heating was continued for 0.5 h, then the heating was stopped, and the sample was cooled to room temperature. After distillation with a Kjeldahl nitrogen determining instrument, titration was performed with 0.1 mol/L hydrochloric acid, and the calculation formula was:

$$W=(V1-V0)\times c\times0.014\times6.25\times250\times10000/[10m(100-X)]$$

where W is the content (%) of water soluble protein; V1 is the hydrochloric acid titrated volume (ml) of each sample; V0 is hydrochloric acid titrated volume (ml) of the blank control; C is titration hydrochloric acid concentration (mol/l); and m is the sample weight (g). X is the water content.

Water soluble protein of 186 samples was determined, 3 technical repetitions were performed for per sample, and CV < 3% (when CV is greater than 3%, samples were excluded to ensure the accuracy of the data). The WSPC ranged from 26.75% to 42.21%, with 31%-41% as the center.

3. Establishment of a model for a near infrared spectrum of water soluble protein in soybean

In step 1, 950-1650 nm original spectra of 186 samples were obtained, and a near infrared model was constructed with 5 nm as an interval. The spectra show that there are 3 peaks between 1020 nm and 1025 nm, between 1200 nm and 1205 nm and between 1500 nm and 1505 nm respectively (FIG. 1-a and FIG. 1-b).

In order to better understand the near infrared spectrum, analysis was made on principal components of 186 original spectra (141 variables) acquired from the soybean samples. A total of 17 outliers were excluded. According to principal component analysis, 86.1% of spectral variances can be solved by the first four principal components (FIG. 1-c). Results show that the near infrared spectrum can be used to establish a model for predicting a WSPC.

Since the wavelength of near infrared electromagnetic radiation is equivalent to the particle size of biological samples, the nonlinearity introduced by light scattering has a great influence on the spectrum. In order to obtain an optimal regression model, SNV, MSC and SG preprocessing methods are applied respectively or in combination (FIG.

2-b).

$R^2$ and SEP values of PLS regression models obtained by each preprocessing method were calculated respectively, and the results are shown in Table 1. $R^2$ obtained by the SG preprocessing method is in the range of 0.824-0.830, which was higher than $R^2$ preprocessed by MSC, SNV, and MSC+SNV(0.817-0.822) (Table 1). The opposite trend was observed in SEP. Without preprocessing of SG conversion, the SEP value was 1.254-1.270; and the application of SG reduced SEP to 1.223-1.239. Since SG conversion contains different smoothing modes determined by a polynomial degree, a polynomial derivative order of and the number of smooth points, previous studies reported that higher order derivatives often led to better PLS equations.

In our study, the 2nd derivative produced higher $R^2$(0.827-0.830) and lower SEP (1.223-1.232). It is worth noting that the PLS regression results of the raw spectrum without preprocessing are lower than those preprocessed by MSC, SNV and SG (first or 2nd derivative) conversion ($R^2$=0.822 0.830, SEP=1.223 1.257), $R^2$ (0.821) and higher than SEP (1.258).

However, factors of PLS equation derived from the original spectrum is more (4-7) than factors (11) generated by preprocessing (Table 1), indicating that the PLS regression model derived from the original spectrum is over-fitted. RPD is often used as an indicator of the effectiveness of regression models. Under different preprocessing conditions, the PLS model generated by MSC+SG (2nd derivative) has the highest RPD (2.411). This shows that the regression model can distinguish high and low WSPC (WSPC) samples.

Table 1: Comparison between principal component analysis processing and un-processing of spectra of all samples

| S N | Preprocessing method | Predicted value | | | Cross-validation value | | | Deviati on | SD | Validati on (RPD) | Facto rs |
|---|---|---|---|---|---|---|---|---|---|---|---|
| | | Slop e | Coefficient of determinati on $R^2$ | RMS EP | Slop e | coefficient of determinati on $R^2$ | Internal cross-validati on RMSEP | | | | |
| 1 | Raw spectra without processing | 0.85 7 | 0.857 | 1.109 | 0.84 0 | 0.821 | 1.258 | (0.012) | 2.94 9 | 2.344 | 11 |
| 2 | SNV | 0.84 6 | 0.840 | 1.177 | 0.83 3 | 0.822 | 1.270 | (0.010) | 2.94 9 | 2.322 | 6 |
| 3 | MSC | 0.83 9 | 0.845 | 1.156 | 0.83 4 | 0.820 | 1.254 | 0.000 | 2.94 9 | 2.352 | 7 |
| 4 | MSC+SNV | 0.84 0 | 0.840 | 1.177 | 0.83 3 | 0.817 | 1.257 | 0.001 | 2.94 9 | 2.346 | 6 |
| 5 | MSC+S.Golay (1st derivative) | 0.85 4 | 0.854 | 1.125 | 0.84 5 | 0.824 | 1.248 | (0.003) | 2.94 9 | 2.363 | 6 |
| 6 | SNV+S.Golay (1st derivative) | 0.85 3 | 0.853 | 1.125 | 0.84 3 | 0.828 | 1.232 | 0.002 | 2.94 9 | 2.394 | 6 |
| 7 | MSC+SNV+S.G olay (1st derivative) | 0.84 6 | 0.846 | 1.153 | 0.83 3 | 0.828 | 1.236 | (0.001) | 2.94 9 | 2.386 | 5 |
| 8 | MSC+S.Golay (2nd derivative) | 0.86 9 | 0.869 | 1.061 | 0.84 1 | 0.829 | 1.223 | (0.002) | 2.94 9 | 2.411 | 4 |
| 9 | SNV+S.Golay (2nd derivative) | 0.85 7 | 0.857 | 1.113 | 0.83 5 | 0.827 | 1.232 | 0.003 | 2.94 9 | 2.384 | 4 |
| 10 | MSC+SNV+S.G olay (2nd derivative) | 0.87 0 | 0.870 | 1.060 | 0.83 8 | 0.830 | 1.229 | 0.000 | 2.94 9 | 2.400 | 5 |

MSC: multiplicative scatter correction; SNV: Standard normal variate; SG: Sabitzky-Golay fitting method; and the 1st derivative and the 2nd derivative are converted by using S.Golay processing.

In summary, the optimal parameters ($R^2$=0.829, SEP=1.223, RPD=2.411) of the WSPC PLS regression model were obtained by using MSC+SG (2nd derivative).

Embodiment 2 Comparison of different methods for extracting water soluble protein

1. Test steps:

Soybean with seed coat removed was crushed to 60 meshes to obtain peeled soybean powder. An aqueous solution was added to some peeled soybean powder to obtain a raw soybean milk solution. The peeled soybean powder was divided into five parts on average, each part being 5 g of soybean powder (0.0001 g). Three of the five parts were respectively mixed with 50 ml of water, and the centrifuged soybean powder solutions were respectively filtered by fast speed filter paper, medium speed filter paper and slow speed filter paper; 50 ml of diethyl ether was added into one part of peeled soybean powder to dissolve fat particles in the soybean powder, shaking and dissolution were performed and then centrifugation was performed to pour out diethyl ether; and the remaining part was not filtered and served as a control group (FIG. 2B).

The water soluble protein was extracted by water solution at room temperature. 50 ml of ultrapure water was added to the defatted soybean sample, and the sample was shaken for 60 min (at 25±2°C) and centrifuged at 1000 r/min for 10 min. Then the supernatant was poured into a 250 ml volumetric flask, and extraction was repeated three times to obtain the water soluble protein extract of soybean. Water was added into a volumetric flask for a constant volume to obtain to-be-tested liquid.

Determination of the WSPC by the kjeldahl nitrogen determining method was performed as follows: 2 g of $CuSO_4 \cdot 5H_2O$ and $K_2SO_4$ mixed catalyst, 10 ml of to-be-tested liquid and 10 ml of concentrated sulfuric acid were sequentially added into a 500 ml digestion tube. The digestion tube was placed in the fume cupboard for heating and digestion, and was heated at a low temperature of 100°C for 0.5 h until black foam disappeared, and the volume of foam in the bottle was controlled not to exceed 2/3 that of the tube; then the temperature was increased to 420°C and heating was performed for 1.5 h; when the digestive fluid was transparent and blue-green, the heating was continued for 0.5 h, then the heating was stopped, and the sample was cooled to room temperature. After distillation with a Kjeldahl nitrogen determining instrument, titration was performed with 0.1 mol/L hydrochloric acid, and the calculation formula was:

$$W=(V1-V0)\times c\times 0.014\times 6.25\times 250\times 10000/[10m(100-X)]$$

where W is the content (%) of water soluble protein; V1 is the hydrochloric acid titrated volume (ml) of each sample; V0 is hydrochloric acid titrated volume (ml) of the blank control; C is titration hydrochloric acid concentration (mol/l); and m is the sample weight (g). X is the water content.

2. Experimental results

Based on the water soluble protein solutions of soybean extracted by the four extraction methods (FIG. 2C), it was found that the water soluble protein solution defatted by diethyl ether was clearer than the water soluble protein solutions filtered by the fast speed, the medium speed filter paper and the slow speed filter paper, and produced no precipitation after standing for a period of time.

Through the determination of the contents of water soluble protein in the soybean water soluble protein solutions extracted by the four methods, it was found that the water soluble protein solution extracted after defatting by diethyl ether was clearer than the water soluble protein solutions obtained by the other three methods, and its WSPC was higher than those of the water soluble protein filtered by fast speed filter paper, the medium speed filter paper and the slow speed filter paper (FIG. 2E).

The water soluble protein components were tested by SDS-PAGE gel electrophoresis (FIG. 2C) after freeze-drying of the water soluble protein solutions extracted by the four methods. No obvious change was found, and unknown protein components 1 and 2 in UF did not exist in the water soluble protein solution (FIG. 2C).

Embodiment 3 Evaluation of the model for a near infrared spectrum of water soluble protein in soybean

1. Experimental Steps

Near infrared spectrum outliers were deleted, and the WSPC PLS regression model was obtained by MSC+SG (2nd derivative) preprocessing method. 48 soybean varieties with different seed coat colors but yellow seed color were selected to evaluate the constructed near infrared spectrum of water soluble protein of soybean.

First, 48 soybean varieties were peeled and subjected to near infrared spectrum scanning to obtain the predicted values. Then the peeled seeds were crushed and sieved with a 60-mesh sieve; 5 g of soybean powder (0.0001 g) was weighed and evenly mixed with 50 ml of diethyl ether, and the mixed solution was continuously shaken for 30 min at 220 r/min to dissolve fat fully. Then centrifugation was performed for 10 min at 2000 r/min, a supernatant was removed, and the solution was placed in a fume cupboard for 30 min until diethyl ether was volatilized completely. The water soluble protein was extracted by water solution at room temperature. 50 ml of ultrapure water was added to the defatted soybean powder, and the sample was shaken for 60 min (at $25\pm2°C$) and centrifuged at 1000 r/min for 10 min. Then the supernatant was poured into a 250 ml volumetric flask, and extraction was repeated three times, and it was set to a constant volume.

The content of protein in the extracted water soluble protein solution was determined by the kjeldahl nitrogen determining method. The specific process included: sequentially adding 2 g of $CuSO_4\cdot5H_2O$ and $K_2SO_4$ mixed catalyst, 10 ml of water soluble protein extract and 10 ml of concentrated sulfuric acid into a 500 ml digestion tube. The digestion tube was placed in the fume cupboard for heating and digestion, and was heated at a low temperature of 100°C for 0.5 h until black foam disappeared, and the volume of foam in the bottle was controlled not to exceed 2/3 that of the tube; then the temperature was increased to 420°C and heating was performed for 1.5 h; when the digestive fluid was transparent and blue-green, the heating was continued for 0.5 h, then the heating was stopped, and the sample was cooled to room temperature. After distillation with a Kjeldahl nitrogen determining instrument, titration was performed with 0.1 mol/L hydrochloric acid to obtain a chemical value measured by the chemical method.

2. Experimental Result

The determination results of predicted values and chemical values are shown in Table 1. Correlation analysis was performed between the chemical values of 48 soybean varieties and the predicted values of the model, and $R^2$ was 0.8927 (FIG. 3). The prediction result was good, and the model capable of stably, accurately and rapidly predicting the WSPC in soybean was implemented.

Table 2: Predicted values and chemical values of 48 soybean samples with different seed coat colors

| Sample | Chemical value | Predicted value | | | Average of predicted values |
|---|---|---|---|---|---|
| 506 | 29.68485 | 30.917 | 30.381 | 31.184 | 30.82733 |
| 602 | 37.33971 | 38.802 | 38.391 | 39.07 | 38.75433 |
| 824 | 36.59499 | 37.701 | 38.562 | 36.587 | 37.61667 |
| 949 | 36.30644 | 36.605 | 37.684 | 36.973 | 37.08733 |
| 1071 | 41.50427 | 41.707 | 42.122 | 41.717 | 41.84867 |
| 1160 | 37.21858 | 38.688 | 38.682 | 37.449 | 38.273 |
| 1237 | 37.44087 | 36.738 | 39.048 | 37.858 | 37.88133 |
| 1272 | 39.24781 | 40.588 | 41.058 | 41.898 | 41.18133 |
| 1341 | 40.32804 | 38.887 | 40.646 | 38.459 | 39.33067 |
| 1421 | 39.9675 | 40.144 | 39.631 | 40.382 | 40.05233 |
| 2122 | 31.61481 | 32.933 | 32.093 | 32.38 | 32.46867 |
| 2328 | 34.90636 | 34.254 | 33.641 | 34.935 | 34.27667 |
| 2678 | 33.3568 | 30.581 | 31.351 | 29.765 | 30.56567 |
| 2773 | 35.22456 | 36.243 | 37.41 | 36.609 | 36.754 |
| 2798 | 36.74459 | 35.515 | 35.813 | 34.811 | 35.37967 |
| 3022 | 34.92227 | 35.464 | 34.59 | 34.766 | 34.94 |
| 3146 | 41.88673 | 42.473 | 41.254 | 40.886 | 41.53767 |
| 3343 | 40.65478 | 40.819 | 41.563 | 40.722 | 41.03467 |
| 3345 | 42.21018 | 42.52 | 42.408 | 43.263 | 42.73033 |

(continued)

| Sample | Chemical value | Predicted value | | | Average of predicted values |
|---|---|---|---|---|---|
| 17D12801 | 33.77139 | 34.627 | 35.089 | 34.789 | 34.835 |
| 17D13201 | 32.36931 | 34.57 | 34.539 | 33.612 | 34.24033 |
| 17D13402 | 35.31364 | 37.101 | 36.6 | 37.561 | 37.08733 |
| 17D13404 | 38.98028 | 40.248 | 40.045 | 39.291 | 39.86133 |
| 17D15003 | 35.14799 | 37.134 | 36.487 | 36.793 | 36.80467 |
| 17D15004 | 36.87035 | 37.845 | 37.692 | 37.712 | 37.74967 |
| 17D21712 | 34.81225 | 35.429 | 35.172 | 35.333 | 35.31133 |
| 17D22015 | 28.5672 | 30.781 | 29.705 | 30.063 | 30.183 |
| 17D22108 | 27.73533 | 30.922 | 31.14 | 30.63 | 30.89733 |
| 17D22201 | 29.22722 | 31.236 | 31.878 | 30.064 | 31.05933 |
| 17D22408 | 33.39634 | 36.098 | 35.371 | 36.135 | 35.868 |
| 17D22411 | 35.97869 | 38.885 | 39.14 | 40.643 | 39.556 |
| 17D22616 | 32.19251 | 33.621 | 33.775 | 34.12 | 33.83867 |
| 17D23601 | 35.62592 | 36.582 | 36.07 | 36.365 | 36.339 |
| 17D24408 | 27.39914 | 29.708 | 29.533 | 29.268 | 29.503 |
| 17D25212 | 37.36398 | 38.983 | 39.54 | 38.435 | 38.986 |
| 17D25213 | 33.39834 | 35.561 | 36.061 | 35.937 | 35.853 |
| 17D25303 | 37.13935 | 39.357 | 39.651 | 39.531 | 39.513 |
| 17D25317 | 37.07138 | 39.571 | 39 | 38.662 | 39.07767 |
| 17D25408 | 37.68327 | 37.449 | 37.488 | 37.602 | 37.513 |
| Jing 58 | 36.88669 | 37.524 | 38.016 | 35.711 | 37.08367 |
| Pixian Silicao | 37.62779 | 39.491 | 39.714 | 39.798 | 39.66767 |
| Zaojiaodou | 35.95181 | 38.479 | 36.839 | 38.376 | 37.898 |
| Suinong 14 | 33.07015 | 33.547 | 32.612 | 33.257 | 33.13867 |
| Zhongdou 41 | 38.04954 | 40.132 | 38.881 | 38.693 | 39.23533 |
| Zhonghuang 13 | 36.55281 | 39.549 | 38.299 | 39.23 | 39.026 |
| Heikewudou | 39.04406 | 40.045 | 39.946 | 39.273 | 39.75467 |
| Dongnong 36 | 33.58709 | 34.688 | 34.35 | 35.078 | 34.70533 |
| Heidou (ZDD09351) | 39.57869 | 40.157 | 40.276 | 40.085 | 40.17267 |

The foregoing descriptions are only preferred implementations of the present invention. It should be noted that for a person of ordinary skill in the art, several improvements and modifications may further be made without departing from the principle of the present invention. The application is only limited by the scope of the appended claims.

## Claims

1. A method for determining a water soluble protein content (WSPC) in soybean by near infrared spectroscopy (NIRS), comprising the following steps:

    (1): peeling off seed coat of to-be-tested soybean to obtain peeled soybean;
    (2): scanning the peeled soybean with a near infrared spectrometer to obtain an original spectrum, wherein the wavelength range of the original spectrum is 950-1650 nm; and

(3): substituting the original spectrum of the soybean into a model for predicting a WSPC in soybean to obtain the WSPC in soybean;

wherein the construction of the model for predicting a WSPC in soybean comprises:

S1: processing each soybean sample according to steps (1) to (2) to obtain an original spectrum;

S2: defatting the soybean sample processed in step S1, determining the WSPC by a chemical method, and recording the content as a measured value; wherein the defatting comprises: crushing the scanned peeled soybean sample, shaking and mixing with diethyl ether, centrifuging to remove a supernatant, and volatilizing the diethyl ether to obtain the defatted soybean sample; and the chemical method for determining the WSPC in soybean comprises the following steps:

A1: mixing the defatted soybean sample with water, performing shaking extraction, and centrifuging to obtain a supernatant to obtain a water soluble protein extract; and

A2: using a Kjeldahl nitrogen determining method to determine the content of protein in the water soluble protein extract to obtain the WSPC in the soybean sample, and recording the content as a measured value;

S3: preprocessing the original spectrum obtained in step S1 by MSC+SG, and establishing a quantitative analysis model by performing partial least squares regression on the preprocessed spectrum;

S4: using the quantitative analysis model established in step S3 to test the soybean sample, and recording an obtained value as a predicted value; and

S5: by comparing the predicted value with the measured value, calculating a standard root mean square error (RMSE) and a correlation coefficient of various quantitative analysis models that may be established in step S3, and selecting an optimal standard RMSE and correlation coefficient of the various quantitative analysis models to obtain the model for predicting a WSPC in soybean.

2. The method according to claim 1, wherein in step (2), the peeled soybean is repeatedly scanned three times, and an average spectrum is calculated as the original spectrum.

3. The method according to claim 1, wherein in step S2, a ratio of the weight of the peeled soybean to the volume of the diethyl ether is (2-10) g:(30-80) ml.

4. The method according to claim 1, wherein in step S2, a shaking and mixing speed is 180- 300 r/min, and the shaking and mixing time is 20-40 min.

5. The method according to claim 1, wherein in step S2, a centrifuging speed is 1500-2500 r/min and the centrifuging time is 8-15 min.

6. The method according to claim 1, wherein in step A1, the operation of mixing with water until centrifugation is implemented is repeated 2-3 times, and the obtained supernatants are combined to obtain the water soluble protein extract.


**Patentansprüche**

1. Verfahren zur Bestimmung des Gehalts an wasserlöslichem Protein (WSPC) in Sojabohnen durch Nahinfrarotspektroskopie (NIRS), umfassend die folgenden Schritte:

(1): Abschälen der Samenschale der zu testenden Sojabohne, um geschälte Sojabohne zu erhalten;

(2): Abtasten der geschälten Sojabohne mit einem Nahinfrarot-Spektrometer, um ein Originalspektrum zu erhalten, wobei der Wellenlängenbereich des Originalspektrums 950-1650 nm beträgt; und

(3): Einsetzen des ursprünglichen Spektrums der Sojabohne in ein Modell zum Vorhersagen eines WSPC in Sojabohne, um den WSPC in Sojabohne zu erhalten;

wobei die Konstruktion des Modells zum Vorhersagen eines WSPC in Sojabohnen Folgendes umfasst:

S1: Verarbeiten jeder Sojabohnenprobe gemäß den Schritten (1) bis (2), um ein Originalspektrum zu erhalten;

S2: Entfetten der in Schritt S1 verarbeiteten Sojabohnenprobe, Bestimmen des WSPC durch ein chemisches Verfahren und Aufzeichnen des Gehalts als Messwert; wobei das Entfetten umfasst: Zerkleinern der gescannten geschälten Sojabohnenprobe, Schütteln und Mischen mit Diethylether, Zentrifugieren, um einen Überstand zu entfernen, und Verflüchtigen des Diethylethers, um die entfettete Sojabohnenprobe zu erhalten; und das chemische Verfahren zur Bestimmung des WSPC in Sojabohnen umfasst die folgenden Schritte:

A1: Mischen der entfetteten Sojabohnenprobe mit Wasser, Durchführen einer Schüttelextraktion und Zentrifugieren, um einen Überstand zu erhalten, um einen wasserlöslichen Proteinextrakt zu erhalten; und

A2: Verwendung eines Kjeldahl-Stickstoffbestimmungsverfahrens zur Bestimmung des Gehalts an Protein im wasserlöslichen Proteinextrakt, um den WSPC in der Sojabohnenprobe zu erhalten, und Aufzeichnen des Gehalts als Messwert;

S3: Vorverarbeiten des in Schritt S1 erhaltenen ursprünglichen Spektrums durch MSC+SG und Erstellen eines quantitativen Analysemodells durch Durchführen einer Regression der partiellen kleinsten Quadrate an dem vorverarbeiteten Spektrum;

S4: Verwendung des in Schritt S3 erstellten quantitativen Analysemodells zum Testen der Sojabohnenprobe und Aufzeichnung eines erhaltenen Werts als Vorhersagewert; und

S5: durch Vergleichen des vorhergesagten Werts mit dem gemessenen Wert, Berechnen eines Standard-Root-Mean-Square-Error (RMSE) und eines Korrelationskoeffizienten verschiedener quantitativer Analysemodelle, die in Schritt S3 erstellt werden können, und Auswählen eines optimalen Standard-RMSE und Korrelationskoeffizienten der verschiedene quantitative Analysemodelle, um das Modell zur Vorhersage eines WSPC in Sojabohnen zu erhalten.

2. Verfahren nach Anspruch 1, wobei in Schritt (2) die geschälte Sojabohne dreimal wiederholt abgetastet wird und ein mittleres Spektrum als das ursprüngliche Spektrum berechnet wird.

3. Verfahren nach Anspruch 1, wobei in Schritt S2 ein Verhältnis des Gewichts der geschälten Sojabohne zum Volumen des Diethylethers (2-10) g:(30-80) ml beträgt.

4. Verfahren nach Anspruch 1, wobei in Schritt S2 eine Schüttel- und Mischgeschwindigkeit 180-300 U/min beträgt und die Schüttel- und Mischzeit 20-40 min beträgt.

5. Verfahren nach Anspruch 1, wobei in Schritt S2 eine Zentrifugiergeschwindigkeit 1500-2500 U/min beträgt und die Zentrifugierzeit 8-15 min beträgt.

6. Verfahren nach Anspruch 1, wobei in Schritt A1 der Vorgang des Mischens mit Wasser bis zur Durchführung der Zentrifugation 2-3-mal wiederholt wird und die erhaltenen Überstände kombiniert werden, um den wasserlöslichen Proteinextrakt zu erhalten.


**Revendications**

1. Procédé de détermination d'une teneur en protéines solubles dans l'eau (WSPC) dans le soja par spectroscopie proche infrarouge (NIRS), comprenant les étapes suivantes :

(1) : décollement du tégument de la graine de soja à tester pour obtenir une graine de soja pelée ;

(2) : balayage de la graine de soja pelée avec un spectromètre proche infrarouge pour obtenir un spectre original, dans lequel la gamme de longueurs d'onde du spectre original est de 950 à 1650 nm ; et

(3): substituer le spectre d'origine du soja dans un modèle pour prédire un WSPC dans le soja afin d'obtenir le WSPC dans le soja ;

dans lequel la construction du modèle pour prédire un WSPC dans le soja comprend les étapes consistant à :

S1: traiter chaque échantillon de soja selon les étapes (1) à (2) pour obtenir un spectre original ;

S2 : dégraisser l'échantillon de graines de soja traité à l'étape S1, déterminer le WSPC par un procédé chimique et enregistrer la teneur en tant que valeur mesurée ; dans lequel le dégraissage comprend les étapes consistant à : écraser l'échantillon de soja décortiqué scanné, secouer et mélanger avec de l'éther diéthylique, centrifuger pour éliminer un surnageant et volatiliser l'éther diéthylique pour obtenir l'échantillon de soja dégraissé ; et le procédé chimique de détermination du WSPC dans le soja comprend les étapes consistant à :

A1: mélanger l'échantillon de graines de soja dégraissées avec de l'eau, effectuer une extraction par agitation et une centrifugation pour obtenir un surnageant afin d'obtenir un extrait protéique soluble dans l'eau ; et

A2 : utiliser une méthode Kjeldahl de détermination de l'azote pour déterminer la teneur en protéine dans l'extrait de protéine soluble dans l'eau pour obtenir le WSPC dans l'échantillon de soja, et enregistrer la teneur en tant que valeur mesurée ;

S3 : prétraiter le spectre original obtenu à l'étape S1 par MSC+SG, et établir un modèle d'analyse quantitative en effectuant une régression des moindres carrés partiels sur le spectre prétraité ;

S4 : utiliser le modèle d'analyse quantitative établi à l'étape S3 pour tester l'échantillon de graines de soja, et enregistrer une valeur obtenue en tant que valeur prédite ; et

S5 : en comparant la valeur prédite à la valeur mesurée, calculer une erreur quadratique moyenne standard (RMSE) et un coefficient de corrélation de divers modèles d'analyse quantitative qui peuvent être établis à l'étape S3, et sélectionner une RMSE standard et un coefficient de corrélation optimaux de divers modèles d'analyse quantitative pour obtenir le modèle de prédiction d'un WSPC dans le soja.

2. Procédé selon la revendication 1, dans lequel à l'étape (2), la graine de soja pelée est balayée trois fois de manière répétée, et un spectre moyen est calculé comme le spectre d'origine.

3. Procédé selon la revendication 1, dans lequel à l'étape S2, un rapport du poids de la graine de soja pelée au volume de l'éther diéthylique est de (2-10) g : (30-80) ml.

4. Procédé selon la revendication 1, dans lequel à l'étape S2, une vitesse d'agitation et de mélange est de 180 à 300 tr/min, et le temps d'agitation et de mélange est de 20 à 40 min.

5. Procédé selon la revendication 1, dans lequel à l'étape S2, une vitesse de centrifugation est de 1500-2500 tr/min et le temps de centrifugation est de 8-15 min.

6. Procédé selon la revendication 1, dans lequel à l'étape A1, l'opération de mélange avec de l'eau jusqu'à ce que la centrifugation soit mise en oeuvre est répétée 2 à 3 fois, et les surnageants obtenus sont combinés pour obtenir l'extrait protéique soluble dans l'eau.

EP 3 734 254 B1

Wavelength (nm)

FIG. 1-a

Wavelength (nm)

FIG. 1-b

17

FIG. 1-c

FIG. 2

(A) Soybean milk

Soluble protein components (α/α', A, Lox isozyme/β-amylase/phytohemagglutinin)

Protein particle polymer — Core part (β/B) 40~200nm / Surface part (α/α'/A)

Fat particles (oleosin/phospholipid/triglyceride) 100-2000nm

Fat particle polymers (α/α'/ A, β/B) 2000-10000nm

Insoluble soybean powder residue <250μm

α/α'  β  7S  • Phytohemagglutinin
• A  • B  11S  β-amylase  Lox isozyme

(B) Fast speed filter paper (80-120 μm)   Medium speed filter paper (30-50 μm)   Slow speed filter paper (1-3 μm)   Processing by diethyl ether

(C) EAT  FF  MF  SF

(D) UF  EAT  FF  MF  SF

(E) Protein content (%)  CP  EAT  FF  MF  SF

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **YAKUN ZHANG et al.** Rapid determination of soluble protein content for soybean leaves based on near infrared spectroscopy. *Nongye Gongcheng Xuebao = Transactions of the Chinese Society of Agricultural Engineering,* 15 September 2018, vol. 34, ISSN 1002-6819, 187-193
- **WEIGUO LU et al.** Identification of the quantitative trait loci (QTL) underlying water soluble protein content in soybean. *Theoretical and Applied Genetics ; International Journal of Plant Breeding Research,* 07 October 2012, vol. 126 (2), ISSN 0040-5752, 425-433
- *Measurement of water soluble protein,* 426-427
- Measurement of protein content in sesame by near-infrared spectroscopy technique. **WANG JIN-SHUI et al.** NEW TECHNOLOGY OF AGRICULTURAL ENGINEERING (ICAE), 2011 INTERNATIONAL CONFERENCE ON. IEEE, 27 May 2011, 1092-1095
- **BAIANU I. et al.** Determination of Soybean Oil, Protein and Amino Acid Residues in Soybean Seeds by High Resolution Nuclear Magnetic Resonance (NMRS) and Near Infrared (NIRS). *Nature Precedings,* 02 April 2012, http://www.nature.com/articles/npre.2012.7053.1